# NEUE EUROPÄISCHE PATENTSCHRIFT

(11) **EP 1 163 883 B2**
(45) Veröffentlichungstag und Bekanntmachung des Hinweises auf die Entscheidung über den Einspruch: **25.09.2013**
(45) Hinweis auf die Patenterteilung: 05.07.2006
(21) Anmeldenummer: 01113410.3
(22) Anmeldetag: 01.06.2001
(51) Int. Cl.: A61B 17/22

(54) **Vorrichtung zum Entfernen von Körpersteinen mit einem intrakorporalen Lithotripter**
Intracorporeal lithotripter for removing calculi
Lithotriteur intracorporelle pour évacuer des concrétions calcaires

(30) Priorität: 15.06.2000 DE 10029581
(43) Veröffentlichungstag der Anmeldung: 19.12.2001
(73) Patentinhaber: Ferton Holding SA, 2800 Delémont (CH)
(72) Erfinder: Menne, Andreas, Dr., 88709 Meersburg (DE); Hirt, Joachim, 78465 Konstanz (DE)
(74) Vertreter: Müller Schupfner & Partner

(56) Entgegenhaltungen:
- EP-A- 0 421 285
- DE-A- 3 625 749
- DE-A- 19 618 972
- DE-A1- 3 520 133
- DE-B2- 2 412 690
- DE-C2- 3 429 487
- DE-C2- 3 712 010
- US-A- 4 827 911
- US-A- 5 906 623
- PSCHYREMBEL 1998: 'Klinisches Wörterbuch', Bd. 258, 1998, WALTER DE GRUYTER
- 'Privatgutachten', 14 Dezember 2000, PROF. DR. H. ERMERT vol. RUHR-UNIVERSITÄT BOCHUM
- AUSZÜGE AUS: 'Fachlexikon ABC Technik', 1983, VERLAG HARRI DEUTSCH
- PNEUMATISCHE VERSUS ELEKTROKINETISCHE: 'ureteroskopische Lithotripsie', 1996, R. VORREUTHER ET AL vol. GEORG THIEME VERLAG: 'Aktuelle urologie 27', Seiten 306 - 310
- VERFAHREN ZUR STEINZERSTÖRUNG IN DEN: 'ableitenden Harnwegen', 1973 vol. M. SAKULIN ET AL: 'Elektrotechnik und Maschinenbau', Seiten 156 - 163
- AUSZUG AUS KATALOG: ' Karl Storz Kaltlicht Endoskope', 1982, INSTRUMENTE
- AUSZUG AUS J.R. FREDERICK: 'Ultrasonic Engineering', 1965, JOHN WILEY & SONS
- AUSZUG AUS BUCH: 'Extracorporeal Shock Waves in', ORTHOPAEDICS, ISBN 3-540-63092-9
- ISBN 978-3-540-34, ISSN 1 02-4 vol. EINSATZ VON STOSSWELLEN IN DER MEDIZIN
- AUSZUG AUS DIN-TASCHENBUCH 22: 'Einheiten und Begriffe für', PHYSIKALISCHE GRÖSSEN

## Beschreibung

Die Erfindung bezieht sich auf eine Vorrichtung zum Entfernen von Körpersteinen mit einem intrakorporalen Lithotripter gemäß dem Oberbegriff des Anspruches 1.

Für die Entfernung von Körpersteinen aus Körperhöhlen ist es bei Überschreitung einer für einen natürlichen Abgang noch ausreichenden Steingröße regelmäßig erforderlich, die Körpersteine zunächst zu zerkleinern, wobei die Zerkleinerung in kleine, spontan abgangsfähige oder direkt aus dem Körper ausspülbare Partikel vorgenommen wird. Die Zerkleinerung der Körpersteine wird dabei durch mechanische Druck- und Zugspannungen vorgenommen, die bei der intrakorporalen Lithotripsie mit dem distalen Ende einer als Wellenleiter dienenden Metallsonde auf die Körpersteine ausgeübt werden. Solche Spannungen führen zu einem Absprengen von Fragmenten aus der Oberfläche eines Steines und bewirken schließlich dessen Zertrümmerung. Für diese Steinzertrümmerung existiert allgemein das Problem einer geeigneten Energieübertragung mit besonderer Beachtung einer Vermeidung von Nebeneffekten auf das biologische Gewebe, das für die Steinzertrümmerung daher nicht als ein Widerlager dienen sollte.

Zur Durchführung einer intrakorporalen Lithotripsie ist aus der EP 0 421 285 B1 eine Vorrichtung bekannt, bei welcher eine Metallsonde oder Sonotrode durch einen elektrisch angesteuerten Ultraschallwandler zu longitudinalen Schwingungen angeregt wird. Mit dem distalen Ende der in den Arbeitskanal eines Endoskops eingesetzten Sonde kann daher die Zertrümmerung eines Körpersteins veranlaßt werden. Der Ultraschallwandler ist dabei mit zwei piezokeramischen Scheiben ausgebildet, die zwischen einem Reflektor und einem Horn verspannt sind. Die beiden Scheiben werden für eine periodische Schwingungsanregung der Sonotrode durch eine Schaltungsanordnung angesteuert, die aus einem spannungsgesteuerten Oszillator besteht, dessen Ausgangssignal über einen Ausgangsverstärker und einen Ausgangsübertrager an die beiden piezokeramischen Scheiben angeliefert wird. Die Schaltungsanordnung umfaßt daneben noch einen Phasenkomparator, der die Phasen der Ausgangsspannung und des Ausgangsstroms des Ausgangsübertragers vergleicht sowie eine Regelspannung zur Steuerung des Oszillators erzeugt. Mit einer Vorrichtung dieser Ausbildung lassen sich die Körpersteine in aller Regel zu sehr feinen Fragmenten zertrümmern. Für die Feinfragmente wird dabei eine Partikelgröße erhalten, die ein meistens problemloses Absaugen durch einen axialen Hohlraum der Sonde hindurch bis hin zu ihrem proximalen Ende erlaubt, an welchem ein durch den Ultraschallwandler hindurchgeführter Sauganschluß zur Wirkung gebracht ist. Die Steinzertrümmerung mittels solcher Ultraschall-Lithotripter ist jedoch relativ zeitaufwändig, da sich mit dem distalen Ende der hohlen Sonotrode bei den für eine gewebeschonende Steinbearbeitung üblichen Ultraschallfrequenzen von etwa 20 bis 25 kHz und Amplituden der Sondenspitze bis etwa 50 µm die Steinzertrümmerung nur reichlich langsam fortführen läßt. Erschwerungen ergeben sich dabei auch bei den härteren Körpersteinen, die sich bei den Ultraschallfrequenzen und Amplituden dieser Größenordnung nicht spontan abschaben lassen, sodaß ihre Bearbeitung entweder sehr lange dauert oder überhaupt nicht möglich ist.

Aus der EP 0 317 507 B1 ist ein Lithotripter bekannt, bei welchem das proximale Ende einer Metallsonde mit einem pneumatisch angetriebenen Schlagteil beaufschlagt wird, um mit einer dadurch erhaltenen Stoßenergie eine die Metallsonde bis hin zu ihrem distalen Ende durchlaufende Stoß- bzw. Druckwelle zu erzeugen. Mit dieser Stoß- bzw. Druckwelle wird auf einen Körperstein eingewirkt, wobei mit der Sondenspitze eine Berührung mit den Steinen eingehalten wird. Solche Stoßwellen-Lithotripter, die bei anderen Ausführungen auch einen elektrischen Antrieb des Schlagteils aufweisen können, ergeben allgemein einen relativ einfachen Geräteaufbau bei ebenfalls sehr guten Fragmentierungsleistungen auch an härteren Körpersteinen. Bei diesen Lithotriptern muß jedoch die Steinzertrümmerung bis hin zu einer absaugfähigen Partikelgröße immer noch relativ zeitaufwändig fortgeführt werden.

Der Erfindung liegt die Aufgabe zugrunde, eine Vorrichtung der eingangs genannten Art derart auszubilden, daß damit unter Berücksichtigung der Vor- und Nachteile dieser bekannten Verfahren eine flexiblere Steinzertrümmerung durchführbar ist.

Diese Aufgabe wird erfindungsgemäß gelöst mit einer Vorrichtung der durch den Anspruch 1 angegebenen Ausbildung, die mit den Merkmalen der weiteren Ansprüche eine vorteilhafte Gestaltungsmöglichkeit erfährt.

Bei der erfindungsgemäßen Vorrichtung werden somit die Arbeitsprinzipien eines herkömmlichen Ultraschall-Lithotripters und eines herkömmlichen Stoßwellen-Lithotripters in einem einzigen Gerät zusammengeführt. Das Überwechseln von einer periodischen Schwingungsanregung der Metallsonde durch den elektrisch angesteuerten Ultraschallwandler zu der mit dem reversibel angetriebenen Schlagteil erhaltenen Ausbildung einer Stoß- bzw. Druckwelle in der Metallsonde ist dabei mit einfachsten Mitteln erreichbar. Von besonderem Vorteil ist, daß für beide Arbeitsprinzipien eine gleich ausgebildete Metallsonde verwendet werden kann, auch bsp. mit der bevorzugten Ausbildung einer rohrförmigen Metallsonde, sodaß über den Hohlraum der Sonde eine Verbindung mit einem äußeren Sauganschluß für ein spontanes Absaugen der bei der Steinzertrümmerung anfallendert Fragmente ermöglicht wird. Es besteht deshalb eine nahezu ideale Voraussetzung zur Durchführung einer variablen und gleichzeitig optimalen Steinzertrümmerung.

Die Steinzertrümmerung kann daneben noch weiter optimiert werden, wenn für die über den Ultraschallwandler elektrisch angesteuerte Schwingunsanregung der Metallsonde auch noch eine Umschaltmöglichkeit zwischen einer periodischen Schwingungsanregung und einer impulsförmigen Schwingungsanregung der Metallsonde vorgesehen ist. Diese weitere Umschaltmöglichkeit ist auf einfache Weise dann realisierbar, wenn zwei getrennte Schaltungsanordnungen vorgesehen sind, die dann über einen Umschalter in eine alternative Anschlußverbindung mit dem Ultraschallwandler einschaltbar sind.

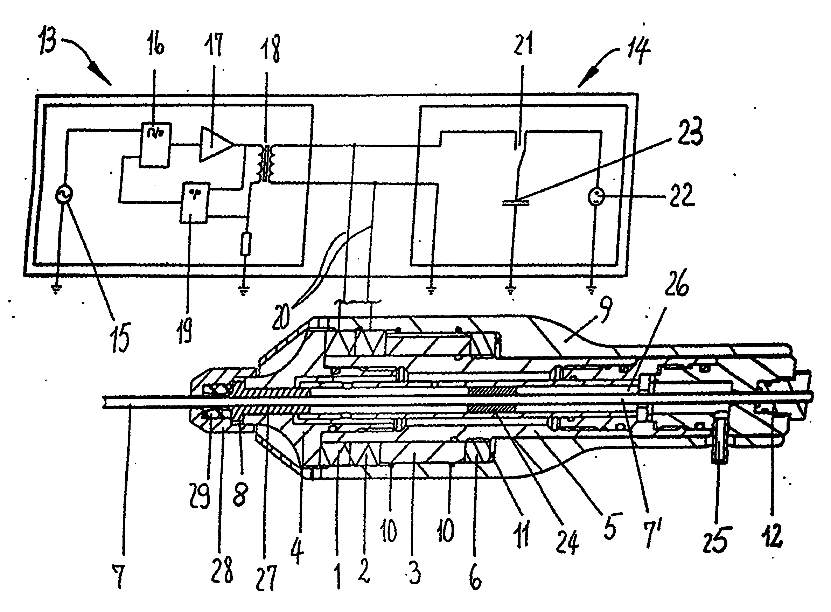

Ein Ausführungsbeispiel der erfindungsgemäßen Vorrichtung ist in der Zeichnung schematisch dargestellt und wird nachfolgend näher erläutert.

Die erfindungsgemäße Vorrichtung besteht aus einem Ultraschallwandler, der als ein piezoelektrischer Wandler mit zwei piezokeramischen Scheiben 1 und 2 mit einer Anordnung zwischen einem Reflektor 3 und einem Horn 4 ausgebildet ist. Die aus diesen Bauteilen bestehende Anordnung des Ultraschallwandlers wird durch eine hohle Spannschraube 5 zusammengehalten, welche die beiden keramischen Scheiben 1, 2 und den Reflektor 3 axial durchsetzt und durch eine Schraubmutter 6 gegen die Anordnung anziehbar ist. Mit dem Horn 4 ist eine rohrförmige Metallsonde bzw. Sonotrode 7 an einem Schraubansatz 8 lösbar verbunden, sodaß sich der Hohlraum der Spannschraube 5 in dem Hohlraum der Metallsonde bzw. Sonotrode 7 axial fortsetzt.

Die mit der Spannschraube 5 zusammengehaltene und gegeneinander verspannte Anordnung der einzelnen Bauteile des Ultraschallwandlers ist innerhalb eines Gehäuses 9 untergebracht. Durch eine zweiteilige Ausbildung dieses Gehäuses mit einer Teilungsebene im Bereich des Horns 4 ist dabei mit der Spannschraube 5 auch eine elastische Verspannung gegen das Gehäuse 9 erreicht, die durch elastische Stützmittel in der Ausbildung von O-Ringen 10 und einer Dichtmanschette 11 eine Abstützung erfährt. Mit der Spannschraube 5 ist ein äußerer Sauganschluß 12 verbunden, sodaß die Steinfragmente, die bei einer Steinzertrümmerung am distalen Ende der Metallsonde 7 anfallen, über den Hohlraum der Metallsonde 7 und den damit axial fluchtenden Hohlraum der Spannschraube 5 spontan abgesaugt werden können.

Die beiden piezokeramischen Scheiben 1, 2 sind für eine an die Metallsonde 7 weitergeleitete Schwingungserzeugung mit einer elektrischen Ansteuerung verbunden, die mit einer ersten Schaltungsanordnung 13 und einer davon getrennten zweiten Schaltungsanordnung 14 ausgebildet ist. Die Schaltungsanordnung 13 ist für eine periodische Schwingungsanregung der Metallsonde vorgesehen und ist dafür im wesentlichen mit einem spannungsgesteuerten Oszillator 15 ausgebildet, der über einen U/F-Wandler 16 und einen nachgeschalteten Verstärker 17 mit einem Impedanz-Transformator 18 verbunden ist. Der Impedanz-Transformator 18 ist mit dem U/F-Wandler 16 über einen Phasenkomparator 19 rückgekoppelt, welcher die Phasen der Ausgangsspannung und des Ausgangsstroms des Impedanz-Transformators 18 vergleicht und eine Regelspannung zur Steuerung des Oszillators 15 erzeugt. Daher wird für die periodische Schwingunsanregung der Metallsonde 7 über die Anschlußverbindung 20 ein passendes Ausgangssignal an die beiden piezokeramischen Scheiben 1, 2 angeliefert.

Die piezokeramischen Scheiben 1, 2 sind daneben mit der zweiten Schaltungsanordnung 14 verbunden, durch welche eine impulsförmige Schwingungsanregung der Metallsonde 7 erhalten wird. Die Schaltungsanordnung 14 besteht aus einer Spannungsquelle 22 und einem Kondensator 23, der in einer ersten Schaltstellung eines Umschalters 21 durch die Spannungsquelle 22 aufgeladen wird. Der Kondensator 23 wird in einer zweiten Schalterstellung des Umschalters 21 entladen, sodaß eine impulsförmige Schwingungsanregung der Metallsonde erhalten wird. Die Kondensatorladung wird dabei einmalig über die Anschlußverbindung 20 an die beiden piezokeramischen Scheiben 1, 2 weitergeleitet, wobei durch das Ausgangssignal der Schaltungsanordnung 14 eine wesentlich höhere Leistung an den piezoelektrischen Wandler angeliefert werden kann als mit dem Ausgangssignal der für die periodische Schwingungsanregung maßgeblichen Schaltungsanordnung 13. Mit dieser höheren Leistung wird aber eine plötzliche Ausdehnung der beiden piezokeramischen Scheiben 1,2 erhalten, die zu einer einmaligen Druckwellenerzeugung in der Metallsonde 7 und damit zu der Abgabe eines Druckimpulses an dem distalen Ende der Sonde führt. Dieser Druckimpuls wird unmittelbar in einen Körperstein eingeleitet, sodaß der Stein augenblicklich zertrümmert wird.

Um für die impulsförmige Schwingungsanregung der Metallsonde optimale Ergebnisse zu erhalten, sollte das Horn 4 des Ultraschallwandlers und die mit ihm verschraubte Metallsonde 7 zweckmäßig aus einem Material mit einer im wesentlichen gleichen akustischen Impedanz bestehen. Als bevorzugte Materialien kommen Edelstahl und Titan in Betracht. Weiterhin sollte das Horn 4 mit einer sich in axialer Richtung etwa auf den Querschnitt des Schraubansatzes 8 der Metallsonde 7 verjüngenden Hüllfläche in der Ausbildung einer Exponentialkurve versehen sein, wobei für die Hüllfläche auch andere Geometrien berücksichtigt werden können. Die mit den elastischen Mitteln 10, 11 bewirkte Abstützung des Ultraschallwandlers an dem umgebenden Gehäuse sollte derart ausgeführt sein, daß sowohl für die periodische wie auch für die impulsförmige Schwingungsanregung der Metallsonde jeweils optimale Arbeitsergebnisse erhalten werden.

Neben der vorstehend detailliert beschriebenen Umschaltmöglichkeit bei der elektrischen Ansteuerung des Ultraschallwandlers zwischen einer periodischen Schwingungsanregung unter Einschaltung der einen Schaltungsanordnung 13 und einer impulsförmigen Schwingungsanregung der Metallsonde unter Einschaltung der weiteren Schaltungsanordnung 14 mit der Umschaltmöglichkeit durch den Umschalter 21 ist die Vorrichtung auch noch umschaltbar auf eine mit einem reversibel angetriebenen Schlagteil 24 gesteuerten Ausbildung einer Stoß- bzw. Druckwelle in der Metallsonde 7. Der Antrieb des Schlagteils 24 kann hydraulisch oder elektrisch oder auch entsprechend der für das Ausführungsbeispiel berücksichtigten Ausbildung pneumatisch erfolgen, wobei dafür ein Druckanschluß 25 an den Hohlraum 26 der Spannschraube 5 vorgesehen ist. Für Einzelheiten des pneumatischen Antriebs des Schlagteils 24 kann auf die EP 0 317 507 B1 verwiesen werden. Für das vorliegende Ausführungsbeispiel der erfindungsgemäßen Vorrichtung ist lediglich noch auf den Unterschied hinzuweisen, daß hier das Schlagteil nicht nur durch die Wand des umgebenden Hohlraums 26 der Spannschraube 5, sondern auch durch eine rückwärtige Verlängerung 7' der hohl ausgeführten Metallsonde 7 axial geführt ist. Das Schlagteil 24 wird jedoch auch bei dieser Anordnung zur Ausübung einer Stoßkraft gegen einen Massekörper 27 der Metallsonde 7 benutzt. Der Massekörper kann dabei eine auf die Sonde im Übergang zu der rückwärtigen Verlängerung 7' aufgeschobene Metallhülse sein, die mit der Sonde fest verbunden ist. Der Massekörper kann für die Übertragung der Stoßkraft des Schlagteils 24 auf die Metallsonde 7 auch eine Ausbildung innerhalb des Hohlraums 26 der Spannschraube 5 erhalten vergleichbar mit einer Anordnung wie beschrieben in der DE 196 18 972 A1, um damit die dort beschriebenen Vorteile der Wirkung eines solchen beschriebenen Massekörpers zu nutzen.

Die Metallsonde 7 ist an einem mit dem Massekörper 27 ausgebildeten Sondenkopf durch eine auf den Schraubansatz 8 aufschraubbare Schraubkappe 28 mit dem Horn 4 des Ultraschallwandlers lösbar verbunden. Der Sondenkopf ist dabei durch ein Dämpfungselement 29 gegen die Schraubkappe 28 abgestützt. Über dieses Dämpfungselement wird eine feste, kraftschlüssige Verbindung zwischen der Metallsonde 7 respektive dem mit dem Massekörper 27 ausgebildeten Sondenkopf und dem Horn 4 des Ultraschallwandlers erhalten, sobald die Schraubkappe 28 genügend fest gegen das Horn 4 angezogen wird. Diese kraftschlüssige Verbindung zwischen dem Sondenkopf und dem Horn des Ultraschallwandlers wird andererseits gelöst, wenn die mit der Schraubkappe 28 bereitgestellte Schraubverbindung gelockert wird und das Dämpfungselement 29 den Massekörper 27 nicht mehr unmittelbar an der Schraubkappe 28 abstützt. Die Metallsonde 7 kann dann eine begrenzte axiale Relativbewegung ausführen. Diese axiale Bewegungsmöglichkeit der Metallsonde 7 wird benötigt, wenn auf die Ausbildung einer Stoß- bzw. Druckwelle in der Metallsonde durch die Ausübung einer Stoßkraft gegen den Massekörper 27 mittels des pneumatisch angetriebenen Schlagteils 24 übergewechselt wird. Die feste, kraftschlüssige Verbindung der Metallsonde 7 mit dem Horn 4 wird dagegen für die über den Ultraschallwandler elektrisch angesteuerte Schwingungsanregung der Metallsonde benötigt.

## Patentansprüche

1. Vorrichtung zum Entfernen von Körpersteinen, umfassend einem intrakorporalen Lithotripter und einen elektrisch angesteuerten Ultraschallwandler, wobei der intrakorporale Lithotripter als Wellenleiter eine Metallsonde aufweist, die für eine mit ihrem distalen Ende veranlaßte Steinzertrümmerung In den Arbeitskanal eines Endoskops eingesetzt und durch den Ultraschallwandler zu longitudinalen Schwingungen angeregt wird,
**dadurch gekennzeichnet, daß** die über den Ultraschallwandler elektrisch angesteuerte Schwingungsanregung der Metallsonde (7) auf eine mit einem reversibel angetriebenen Schlagtell (24) der Vorrichtung gesteuerte Ausbildung einer Stoß- bzw. Druckwelle in der Metallsonde (7) umschaltbar ist.

2. Vorrichtung nach Anspruch 1, **dadurch gekennzeichnet, daß** die über den Ultraschallwandler elektrisch angesteuerte Schwingungsanregung der Metallsonde (7) zwischen einer periodischen Schwingungsanregung und einer impulsförmigen Schwingungsanregung umschaltbar ist.

3. Vorrichtung nach Anspruch 2, **dadurch gekennzeichnet, daß** für die elektrisch angesteuerte impulsförmige Schwingungsanregung der Metallsonde (7) eine von der Schaltungsanordnung (13) für die elektrisch gesteuerte periodische Schwingungsanregung der Metallsonde (7) getrennte Schaltungsanordnung (18) vorgesehen ist, die mit einer Spannungsquelle (22) und mit einem Kondensator (23) gebildet und über einen Umschalter (21) in eine Anschlußverbindung (20) mit dem Ultraschallwandler einschaltbar ist.

4. Vorrichtung nach Anspruch 3, **dadurch gekennzeichnet, daß** der Kondensator (23) in einer ersten Schaltstellung des Umschalters (21) durch die Spannungsquelle (22) der getrennten Schaltungsanordnung (14) aufgeladen wird und für die Anschlußverbindung (20) mit dem Ultraschallwandler in eine zweite Schaltstellung umschaltbar ist.

5. Vorrichtung nach eine der Ansprüche 1 bis 4, **dadurch gekennzeichnet, daß** der Ultraschallwandler mit wenigstens einer piezokeramischen Scheibe (1, 2) gebildet ist, die mit einer Anordnung zwischen einem Reflektor (3) und einem die Metallsonde (7) tragenden Horn (4) durch eine hohle Spannschraube (5) gegeneinander verspannt sind, wobei der Hohlraum (26) der Spannschraube (5) das reversibel angetriebene Schlagteil (24) aufnimmt und das Schlagteil (24) zur Ausübung einer Stoßkraft gegen einen Massekörper (27) der mit dem Horn (4) lösbar verbundenen Metallsonde (7) angeordnet ist.

6. Vorrichtung nach Anspruch 5, **dadurch gekennzeichnet, daß** die Metallsonde (7) an einem mit dem Massekörper (27) ausgebildeten Sondenkopf durch eine Schraubkappe (28) mit dem Horn (4) des Ultraschallwandlers lösbar verbunden ist, wobei der Sondenkopf gegen die Schraubkappe (28) durch ein Dämpfungselement (29) abgestützt ist, welches die Metallsonde (7) mittels der Schraubkappe (28) mit dem Horn (4) des Ultraschallwandlers kraftschlüssig verbinden läßt.

7. Vorrichtung nach Anspruch 5 oder 6, **dadurch gekennzeichnet, daß** die Metallsonde (7) rohrförmig ausgebildet und an ihrem die Stoßkraft des Schlagteils (24) aufnehmenden Sondenkopf mit einer in den Hohlraum (26) der Spannschraube (5) axial vorstehenden rückwärtigen Verlängerung (7') versehen ist, an welche ein äußerer Sauganschluß (12) angeschlossen ist.

8. Vorrichtung nach einem der Ansprüche 1 bis 7, **dadurch gekennzeichnet, daß** das Schlagteil (24) eine zentrale Bohrung aufweist und durch die rückwärtige Verlängerung (7') der Metallsonde (7) sowie an seinem Umfang durch die Wandung des Hohlraumes (26) der Spannschraube (5) axial geführt ist.

9. Vorrichtung nach einem der Ansprüche 5 bis 8, **dadurch gekennzeichnet, daß** der Hohlraum (26) der Spannschraube (5) mit einem Druckanschluß (25) für einen pneumatischen Antrieb des Schlagteils (24) versehen ist.

10. Vorrichtung nach einem der Ansprüche 5 bis 9, **dadurch gekennzeichnet, daß** die mit der Spannschraube (5) gegenseitig verspannte Anordnung der wenigstens einen piezokeramischen Scheibe (1,2), des Reflektors (3) und des die Metallsonde (7) tragenden Horns (4) durch elastische Stützmittel (10, 11) gegen das umgebende Gehäuse (9) abgestützt ist.

11. Vorrichtung nach einem der Ansprüche 5 bis 10, **dadurch gekennzeichnet, daß** das Horn (4) mit einer sich in axialer Richtung etwa auf den Querschnitt eines Schraubansatzes (8) für die Verschraubung der Schraubkappe (28) verjüngenden Hüllfläche in der Ausbildung einer Exponentialkurve versehen ist.

12. Vorrichtung nach einem der Ansprüche 5 bis 11, **dadurch gekennzeichnet, daß** die Metallsonde (7) und das Horn (4) aus einem Material mit einer im wesentlichen gleichen akustischen Impedanz bestehen.

## Claims

1. A device for removing corporeal calculi comprising an intracorporeal lithotripter and an electrically driven ultrasonic transducer, wherein the intracorporeal lithotripter has a metal probe employed as a waveguide whose distal end is inserted into the operating channel of an endoscope and stimulated to produce longitudinal oscillations by the ultrasonic transducer in order to shatter calculi,
**characterized in that** the electrically controlled stimulation of oscillations of the metal probe (7) by the ultrasonic transducer may be switched to forming a shock or pressure wave within the metal probe (7), controlled by a reversibly driven, impacting component (24) of the device.

2. A device according to Claim 1, **characterized in that** the electrically controlled stimulation of oscillations of the metal probe (7) driven by the ultrasonic transducer may be switched between stimulation of periodic oscillations and stimulation of pulsed excursions.

3. A device according to Claim 2, **characterized in that** circuitry (14) that is separate from the circuitry (13) of the electrically controlled stimulation of periodic oscillations of the metal probe (7) has a voltage source (22), is configured using a capacitor (23), and may be switched into a connection (20) to the ultrasonic transducer by a selector switch (21) which is provided for the electrically controlled stimulation of pulsed oscillations of the metal probe (7)

4. A device according to Claim 3, **characterized in that**, in a first switching position of the selector switch (21), the capacitor (23) is charged by the voltage source (22) of the separate circuitry (14) and may be switched to a second switching position in order to generate the connection (20) to the ultrasonic transducer.

5. A device according to any of Claims 1 to 4, **characterized in that** the ultrasonic transducer is configured using at least one piezoelectric disk (1, 2) that, together with an arrangement between a reflector (3) and a horn (4) supporting the metal probe (7), are clamped together by a hollow setscrew (5), where the cavity (26) of the setscrew (5) accommodates the reversibly driven impacting component (24) and the impacting component (24) is arranged for exerting an impulsive force on a tamper (27) arranged on the metal probe (7) that is attached to the horn (4) such that it may be withdrawn therefrom.

6. A device according to Claim 5, wherein the metal probe (7) on a probe head configured with the tamper (27) is attached to the horn (4) of the ultrasonic transducer by a threaded cap (28) such that it may be withdrawn therefrom, where the probe head is supported against the threaded cap (28) by a damping component (29), which allows attaching the metal probe (7) to the horn (4) of the ultrasonic transducer using the threaded cap (28) such that the former will be held firmly in place in the latter.

7. A device according to Claim 5 or 6, **characterized in that** the metal probe (7) is tubular and provided with a rear, axial extension (7'), to which an external suction fitting (12) is attached, protruding into the cavity (26) of the setscrew (5) on its probe head which receives the impulsive force exerted by the impacting component (24).

8. A device according to any of Claims 1 to 7, **characterized in that** the impacting component (24) has a central bore and is axially guided on the rear extension (7') of the metal probe (7), as well as radially guided by the wall of the cavity (26) of the setscrew (5).

9. A device according to any of Claims 5 to 8, **characterized in that** cavity (26) of the setscrew (5) is provided with a pressure fitting (25) for connecting a pneumatic drive for the impacting component (24).

10. A device according to any of Claims 5 to 9, **characterized in that** the arrangement of the at least one piezoelectric disk (1, 2), the reflector (3), and the horn (4) supporting the metal probe (7), which are clamped together by the setscrew (5), is supported on the surrounding housing (9) by elastic supports (10, 11).

11. A device according to any of Claims 5 to 10, **characterized in that** the horn (4) is provided with an outer surface having the shape of an exponential curve that axially tapers to approximately the lateral dimensions of a threaded abutment (8) for the threaded cap (28).

12. A device according to any of Claims 5 to 11, **characterized in that** the metal probe (7) and the horn (4) consist of materials having essentially identical acoustic impedances.

## Revendications

1. Dispositif d'élimination de calculs rénaux à l'aide d'un lithotripteur intra-corporel et un transducteur à ultrasons à commande électrique; dans lequel le lithotripteur intra-corporel présente comme guide d'ondes une sonde métallique introduite dans le cathéter d'un endoscope en vue d'une fragmentation du calcul provoquée par son extrémité distale et excitée en oscillations longitudinales par le transducteur à ultrasons, **caractérisé en ce que** l'excitation oscillatoire de la sonde métallique (7) commandée électriquement par le transducteur à ultrasons peut être basculée pour former, sur la commande d'une pièce battante (24) du dispositif entraînée de façon réversible, une onde de choc ou de pression dans la sonde métallique (7).

2. Dispositif selon la revendication 1, **caractérisé en ce que** l'excitation oscillatoire de la sonde métallique (7) commandée électriquement par le transducteur à ultrasons peut être basculée entre une excitation oscillatoire périodique et une excitation oscillatoire pulsée.

3. Dispositif selon la revendication 2, **caractérisé en ce qu'**un dispositif commutateur (14), distinct du dispositif commutateur (13) prévu pour l'excitation oscillatoire périodique à commande électrique de la sonde métallique (7), est prévu pour l'excitation oscillatoire pulsée à commande électrique de la sonde métallique (7), qui est constitué d'une source de tension (22) et d'un condensateur (23) et qui peut être connecté au transducteur à ultrasons dans une liaison de connexion (20) par l'intermédiaire d'un commutateur (21).

4. Dispositif selon la revendication 3, **caractérisé en ce que** le condensateur (23), dans une première position de commutation du commutateur (21), est chargé par la source de tension (22) du dispositif commutateur distinct (14) et peut être basculé dans une seconde position de commutation pour la liaison de connexion (20) au transducteur à ultrasons.

5. Dispositif selon l'une quelconque des revendications 1 à 4, **caractérisé en ce que** le transducteur à ultrasons est constitué d'au moins un disque piézocéramique (1,2), qui est serré contre un autre disque piézocéramique par une vis de serrage creuse (5), à l'aide d'un dispositif situé entre un réflecteur (3) et une corne (4) portant la sonde métallique (7), la cavité (26) de la vis de serrage (5) logeant la pièce battante (24) entraînée de façon réversible et la pièce battante (24) étant disposée pour exercer une force de poussée contre un corps de masse (27) de la sonde métallique (7) reliée de façon amovible à la corne (4).

6. Dispositif selon la revendication 5, **caractérisé en ce que** la sonde métallique (7) est reliée de façon amovible à la corne (4) du transducteur à ultrasons par un couvercle vissé (28), au niveau d'une tête de sonde constituée du corps de masse (27), la tête de sonde étant appuyée contre le couvercle vissé (28) par un élément amortisseur (29) qui met en liaison de force la sonde métallique (7) et la corne (4) du transducteur à ultrasons à l'aide du couvercle vissé (28).

7. Dispositif selon la revendication 5 ou 6, **caractérisé en ce que** la sonde métallique (7) présente la forme d'un tube et est pourvue, au niveau de sa tête de sonde réceptionnant la force de poussée de la pièce battante (24), d'une allonge (7') arrière dépassant en direction axiale dans la cavité (26) de la vis de serrage (5), un embout d'aspiration externe (12) étant raccordé à cette allonge.

8. Dispositif selon l'une quelconque des revendications 1 à 7, **caractérisé en ce que** la pièce battante (24) présente un alésage central et est déplacée en direction axiale par l'allonge (7') arrière de la sonde métallique (7) et, en son pourtour, par la paroi de la cavité (26) de la vis de serrage (5).

9. Dispositif selon l'une quelconque des revendications 5 à 8, **caractérisé en ce que** la cavité (26) de la vis de serrage (5) est pourvue d'un raccord de pression (25) pour un entraînement pneumatique de la pièce battante (24).

10. Dispositif selon l'une quelconque des revendications 5 à 9, **caractérisé en ce que** le dispositif constitué des disques piézocéramiques (1,2) serrés l'un contre l'autre à l'aide de la vis de serrage (5), du réflecteur (3) et de la corne (4) portant la sonde métallique (7) est appuyé contre le boîtier (9) qui l'entoure par des moyens d'appui élastiques (10,11).

11. Dispositif selon l'une quelconque des revendications 5 à 10, **caractérisé en ce que** la corne (4) est pourvue d'une surface enveloppante qui se rétrécit en direction axiale environ jusqu'à la section transversale d'un embout vissé (8) pour le vissage du couvercle vissé (28), pour former une courbe exponentielle.

12. Dispositif selon l'une quelconque des revendications 5 à 11, **caractérisé en ce que** la sonde métallique (7) et la corne (4) sont composées de matériaux d'une impédance acoustique essentiellement identique.
